# EUROPEAN PATENT APPLICATION

(11) **EP 4 725 481 A1**
(43) Date of publication of application: **15.04.2026**
(21) Application number: 24205837.8
(22) Date of filing: 10.10.2024
(51) Int. Cl.: A61K 31/403, A61K 31/416, A61P 31/20, A61P 11/00

(54) **1,2,3,4-TETRAHYDROCARBAZOLE DERIVATIVES FOR USE IN TREATING AN ADENOVIRAL INFECTION**

(71) Applicant: Leibniz-Institut für Virologie, 20251 Hamburg (DE)
(72) Inventor: IP, Wing Hang, 20251 Hamburg (DE); BERTZBACH, Luca, 20251 Hamburg (DE); DOBNER, Thomas, 20251 Hamburg (DE); HOFMANN-SIEBER, Helga, 22417 Hamburg (DE)
(74) Representative: Stüven, Ralf

(57) **Abstract**

The invention relates to derivatives of 1-amino-1,2,3,4-tetrahydrocarbazole as new active agents against adenoviral infection.

## Description

The invention relates to new active agents against adenoviral infection.

Adenovirus infections usually lead to mild, mostly asymptomatic courses with respiratory symptoms in immunocompetent patients. However, in immunosuppressed patients, the viruses can cause life-threatening illnesses that manifest as acute respiratory distress syndrome (ARDS) and can be accompanied by pneumonia, hepatitis, meningitis, diarrhoea, rashes and cystitis. To date, there is no specific drug therapy against human adenovirus infections (see, e.g., Lion T. (2019) Adenovirus persistence, reactivation, and clinical management, FEBS Lett. 593:3571-3582, doi: 10.1002/1873-3468.13576; Saha B, Parks RJ. Recent Advances in Novel Antiviral Therapies against Human Adenovirus. Microorganisms. 2020 Aug 22;8(9):1284. doi: 10.3390/microorganisms8091284; Dodge MJ, MacNeil KM, Tessier TM, Weinberg JB, Mymryk JS. Emerging antiviral therapeutics for human adenovirus infection: Recent developments and novel strategies. Antiviral Res. 2021 Apr; 188:105034. doi: 10.1016/j.antiviral.2021.105034).

Previous attempts to treat adenovirus infections in immunocompromised patients have been based on off-label treatment with intravenous cidofovir (trade name "Vistide", a nucleotide analogue used primarily to treat an eye disease that can be caused by cytomegalovirus, CMC), its derivative brincidofovir (CMX001), or related antiviral agents with a broad spectrum of activity, which, however, can cause serious side effects such as nephrotoxicity and detrimental effects on the gastrointestinal tract (see, e.g. Lion, T. (2019), above). In addition, preliminary experimental data in relation to a few other experimental inhibitors have been published, for example, arsenic trioxide, ATO (Hofmann S, Mai J, Masser S, Groitl P, Herrmann A, Sternsdorf T, Brack-Werner R, Schreiner S. (2020), ATO (Arsenic Trioxide) Effects on Promyelocytic Leukemia Nuclear Bodies Reveals Antiviral Intervention Capacity, Adv Sci (Weinh) 7:1902130, doi: 10.1002/advs.201902130), ivermectin (King CR, Tessier TM, Dodge MJ, Weinberg JB, Mymryk JS. (2020), Inhibition of Human Adenovirus Replication by the Importin α/β1 Nuclear Import Inhibitor Ivermectin, J Virol. 94:e00710-20, doi: 10.1128/JVI.00710-20), and cyclosporine (Böhringer D, Birnbaum F, Reinhard T. (2008) Cyclosporin-A-Augentropfen bei Nummuli nach Adenovirus-Keratokonjunktivitis

[Cyclosporin A eyedrops for keratitis nummularis after adenovirus keratoconjunctivitis], Ophthalmologe 105:592-4, doi: 10.1007/s00347-008-1731-1). These inhibitors have not yet been validated in preclinical studies in the context of adenoviral infections.

There is a need for anti-adenoviral agents, in particular agents suitable for treating adenoviral infections in immunocompromised patients, and/or in patients with keratoconjunctivitis. It is an object of the invention to provide such an anti-adenoviral agent.

For solving the problem, the invention provides a chemical compound having the following general formula I 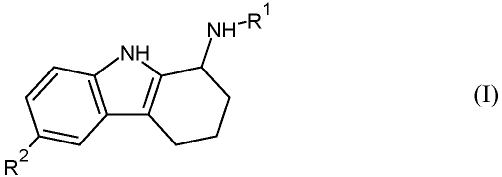 or a salt thereof, wherein
R¹ is selected from the group consisting of the following chemical groups 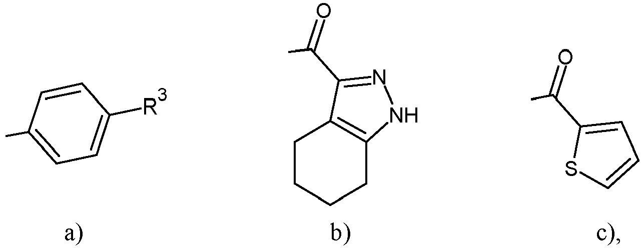
R² is, independently, selected from H, halogen or C₁₋₁₀ alkyl, and
R³ is halogen,
for use as a medicament for treating an adenovirus infection.

The compounds of the invention have been found to be highly active against adenoviral infections, and to have a low cell toxicity. Adenoviruses, in humans most often of types B, C, D, E and F, typically cause respiratory illnesses or keratoconjunctivitis. Although usually associated with a mild course, adenoviral infections can cause severe diseases and even death, in particular in immunocompromised people, i.e. patients with an impaired immunological response (see, for example, Echavarría M. (2008), Adenoviruses in immunocompromised hosts, Clin Microbiol Rev. 21:704-15, doi: 10.1128/CMR.00052-07; Lion T. (2019) Adenovirus persistence, reactivation, and clinical management, FEBS Lett. 593:3571-3582, doi: 10.1002/1873-3468.13576). Manifestations of adenoviral infections in immunocompromised patients may result in disseminated disease including pneumonia, colitis, hepatitis, hemorrhagic cystitis, tubulointerstitial nephritis, and encephalitis.

The term "adenovirus" is used here to refer to a virus of the Adenoviridae family. Adenoviruses are non-enveloped viruses whose genome is in the form of a double-stranded linear DNA in an icosahedral capsid composed of penton and hexon capsomers. The family includes atadenoviruses, aviadenoviruses, ichtadenoviruses, siadenoviruses, testadenovirus and mastadenoviruses, the latter of which occur in mammals and also include human adenoviruses (HAdV), which infect human cells. HAdVs are further subdivided into species (currently A-G) and serotypes, (see e.g. Ghebremedhin B. (2014) Human adenovirus: Viral pathogen with increasing importance, European Journal of Microbiology & Immunology 4(1):26-33. doi:10.1556/EuJMI.4.2014.1.2; Benkő M, Aoki K, Arnberg N, Davison AJ, Echavarría M, Hess M, Jones MS, Kaján GL, Kajon AE, Mittal SK, Podgorski II, San Martin C, Wadell G, Watanabe H, Harrach B, ICTV Report Consortium (2022), ICTV Virus Taxonomy Profile: Adenoviridae 2022, J Gen Virol. 103:001721, doi: 10.1099/jgv.0.001721). The HAdVs of species C includes viruses of serotypes 1, 2, 5 and 6, for example.

The terms "adenoviral infection" or "adenovirus infection" relate to infections caused by adenovirus, i.e. the entry of an adenovirus into a cell or body, e.g., a human cell or body. The terms encompass diseases caused or associated with the infection. Adenoviral infections can, for example, manifest as gastroenteritis, respiratory or urinary tract infections and (kerato)conjunctivitis. For example, adenoviruses of species A cause gastroenteritis, respiratory and urinary tract infections, adenoviruses of species B, C and E cause respiratory infections and (kerato)conjunctivitis, adenoviruses of species D causes (kerato-)conjunctivitis and gastroenteritis, and adenoviruses of species F cause gastroenteritis. "Keratoconjunctivitis refers to an inflammatory process involving the conjunctiva and the superficial cornea.

The term "prodrug" relates to a precursor of a drug, i.e. a compound, which itself is pharmacologically inactive or at least only little active, and undergoes chemical conversion by metabolic processes before becoming an active pharmacological agent. Prodrugs are often used for delivering chemical compounds carrying functional groups which negatively affect bioavailability (e.g. functional groups with negatively charged atoms at physiological pH) and therefore must be masked. The term "prodrug comprising the compound of the invention" relates to a prodrug from which the compound of the invention is produced or released by metabolic processes in vivo, for example in a target cell.

The term "pharmaceutically acceptable carrier" refers to any organic or inorganic, natural or synthetic substance, which can be combined with an active ingredient or a combination of active ingredients to simplify, improve selectivity, effectiveness and/or safety of application, and/or to improve or control release, distribution or targeting of the active ingredient or combination of active ingredients in the body. Examples of such carriers include, but are not limited to organic or inorganic solvents, starch, lactose, mannitol, methylcellulose, talc, gelatin, agar agar, calcium phosphate, magnesium stearate, animal and vegetable fats, higher molecular weight fatty acids, lipids, phospholipids, or higher molecular weight polymers.

The term "pharmaceutically acceptable" means any substantially non-toxic material for mammals, especially humans, which does not substantially affect the effectiveness of the biological activity of the active ingredient. Such materials may include pharmaceutically acceptable concentrations of salts, buffers, preservatives, or the like. Non-limiting examples of pharmaceutically acceptable carriers include magnesium carbonate, magnesium stearate, talc, sugar, lactose, ethanol, glycerol, water, buffer solutions. The pharmaceutical composition may also comprise adjuvants and/or diluents.

The term "pharmaceutically acceptable salt" means any salt of a compound that is substantially non-toxic for mammals, especially humans, and that does not substantially affect the effectiveness of the biological activity of the active ingredient.

The term "halogen" refers to any element of group 17 (VIIa) of the periodic table, i.e. to fluorine, chlorine, bromine, iodine, and astatine, in particular to any of the elements fluorine, chlorine, bromine, and iodine.

For the purposes of the present invention, the indication of a range such as "1-10", for example, is to be understood as meaning that each intermediate value is also disclosed. In the case of an indication which can only relate to integers, such as, for example, a number of C atoms, this means, of course, that only integers are disclosed. Any narrower range from a broader range is also meant to be disclosed by indicating the broader range, the narrower range also including ranges not comprising any of the boundary values of the broader range (e.g. a range of 2-5 from a range of 1-10).

The term "Cₙ-Cₘ" or "Cₙ₋ₘ", where n and m are each positive integers and m is greater than n, means a range indicating the number of carbon atoms of a compound or residue. The expression here expressly includes all integer intermediate values between the range boundaries n and m, in each case independently of one another. The expression "C₁₋₁₀" (n = 1, m = 10) therefore means, for example, a compound, group or residue having 1-10, i.e. 1, 2, 3, 4, 5, 6, 7, 8, 9 or 10 carbon atoms. "C₁₋₁₀" therefore also comprises, for example, "C₂₋₆", i.e. 2, 3, 4, 5 or 6 carbon atoms, or "C₁₋₄", i.e. 1, 2, 3 or 4 carbon atoms, or "C₄₋₉", i.e. 4, 5, 6, 7, 8 or 9 carbon atoms. Correspondingly, the term "C₁₋₁₀ alkyl" means, for example, an alkyl group having 1, 2, 3, 4, 5, 6, 7, 8, 9 or 10 carbon atoms and comprises all combinations of the values of n and m, which lie in the range from n = 1 to m = 10, e.g. C₁₋₃ alkyl C₂₋₅ alkyl or C₃₋₁₀ alkyl.

The compounds of the invention are derivatives of 1-amino-1,2,3,4-tetrahydrocarbazole, or salts of such derivatives. Any salt of a compound of the invention is preferably a pharmaceutically acceptable salt.

The chemical compound for use as a medicament for treating an adenovirus infection according to the invention can be a compound of one of the following formulas Ia-Ic: 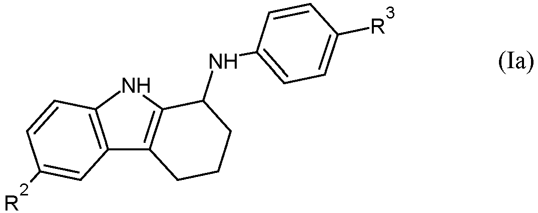 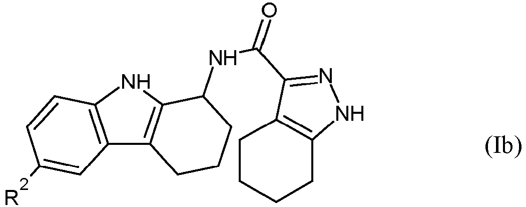 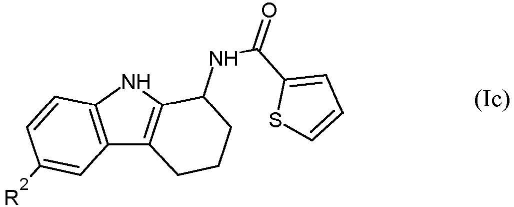 R² and R³ are as defined above.

In a preferred embodiment, the chemical compound for use as a medicament for treating an adenovirus infection according to the invention is a compound of formula I above, wherein R² is H, halogen, or C₁₋₈ alkyl, preferably C₁₋₆, more preferably C₁₋₄ alkyl, C₁₋₃ alkyl or C₁₋₂ alkyl, most preferably methyl. In preferred embodiments R² is either H, methyl or fluorine, F.

In a further preferred embodiment, the chemical compound for use as a medicament for treating an adenovirus infection according to the invention is a compound of formula I above, wherein, R¹ is 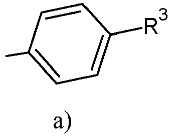 and R³ is Br.

In a particular preferred embodiment, the chemical compound for use as a medicament for treating an adenovirus infection according to the invention is a chemical compound having one of the following general formulas Ia1-Ic1:

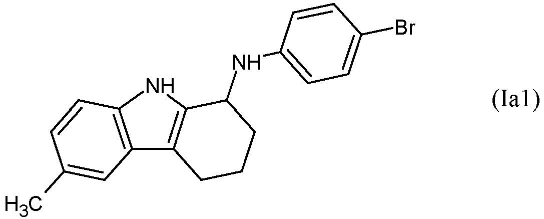

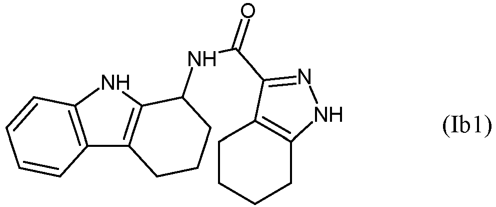

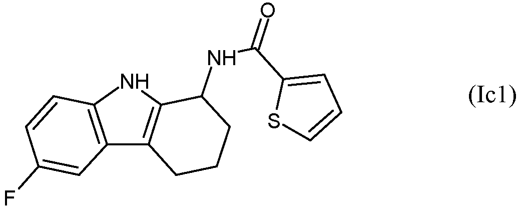

In a still further aspect, the invention relates to a prodrug comprising the compound of the invention, and from which the compound of the invention is released in vivo, for use as a medicament for treating an adenovirus infection. A prodrug of the invention may, for example, be derived from a compound according to formula I by N-acylation, N-Acyloxyalkylation or N-(phosphoryloxy)alkylation at the N atom of the 1,2,3,4-tetrahydrocarbazole structure (see, e.g., Simplicio AL, Clancy JM, Gilmer JF. Prodrugs for amines. Molecules. 2008 Mar 3;13(3):519-47. doi: 10.3390/molecules13030519). Examples of a prodrug of the invention may thus include compounds of formula I substituted, at the N atom of the 1,2,3,4-tetrahydrocarbazole structure, with a suitable functional group, e.g., an acetyl group (-COCH₃), acetyloxyalkyl group (e.g. RCOOCH₂-, R = e.g. alkyl) or a phosphoryloxy methyl group (P(O₃)OCH₂-).

In a still further aspect, the invention relates to a pharmaceutical composition or dosage form for use as a medicament for treating an adenovirus infection comprising a compound according to the invention and a pharmaceutically acceptable carrier. Pharmaceutically acceptable carriers are known to the person skilled in the art and may comprise one or more liquid, semisolid or solid fillers, diluents or other substances suitable for administration to mammals, including humans.

The pharmaceutical composition or dosage form according to the invention may be adapted to any suitable route of administration, e.g. for oral, rectal or parenteral (e.g. intravenous) administration. A preferred pharmaceutical composition or dosage form of the invention is adapted for oral administration, for example a tablet or capsule, or as a solution or suspension for intravenous administration. A pharmaceutical composition or dosage form of the invention can also be a liposome or liposome formulation (see, for example, Schwendener, Reto & Schott, Herbert. (2010), Liposome Formulations of Hydrophobic Drugs, Methods in molecular biology (Clifton, N.J.), 605, 129-38, doi:10.1007/978-1-60327-360-2_8; Olusanya TOB, Haj Ahmad RR, Ibegbu DM, Smith JR, Elkordy AA. Liposomal Drug Delivery Systems and Anticancer Drugs, Molecules 2018, 23(4):907, doi:10.3390/molecules23040907; Bulbake U, Doppalapudi S, Kommineni N, Khan W. Liposomal Formulations in Clinical Use: An Updated Review, Pharmaceutics 2017, 9(2): 12, doi: 1 0.3390/pharmaceutics90200 12). Liposomes, spherical vesicles comprising a bilayer of lipids with an internal aqueous cavity, are known to the skilled person and are particularly useful for the administration of lipophilic compounds.

In a still further aspect, the invention also relates to a method of treatment of an adenovirus infection, the method comprising administering to a patient in need thereof an effective amount of a compound of the invention. In a preferred embodiment of the method of the invention the compound of the invention is in the form of a pharmaceutical composition or dosage form according to the invention, as described above.

The invention is explained in more detail below with reference to the accompanying figures and exemplary embodiments for illustrative purposes only.

### BRIEF DESCRIPTION OF THE DRAWINGS

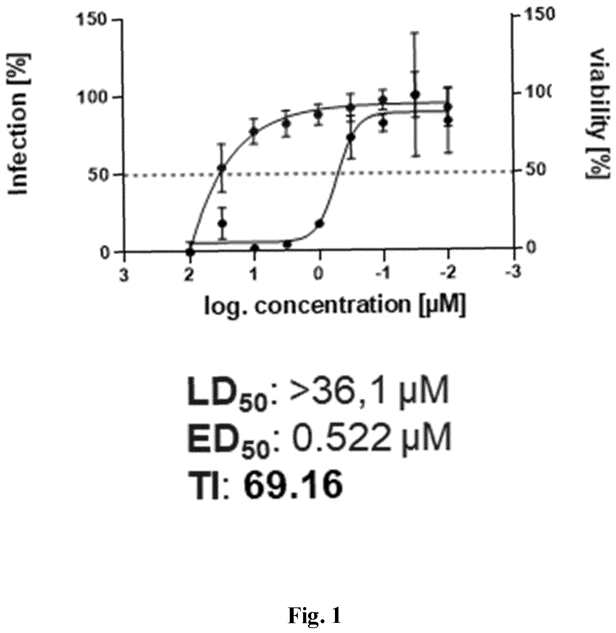

FIG. 1 Dose-response curves for A549 cells infected with HAdV-C5 and treated with varying concentrations of compound #11 (compound of formula 1a1). Lower (sigmoidal) curve: % viability; upper curve: % infection. LD₅₀ = median lethal dose; ED₅₀ = median effective dose; TI = therapeutic index (LD₅₀/ED₅₀).

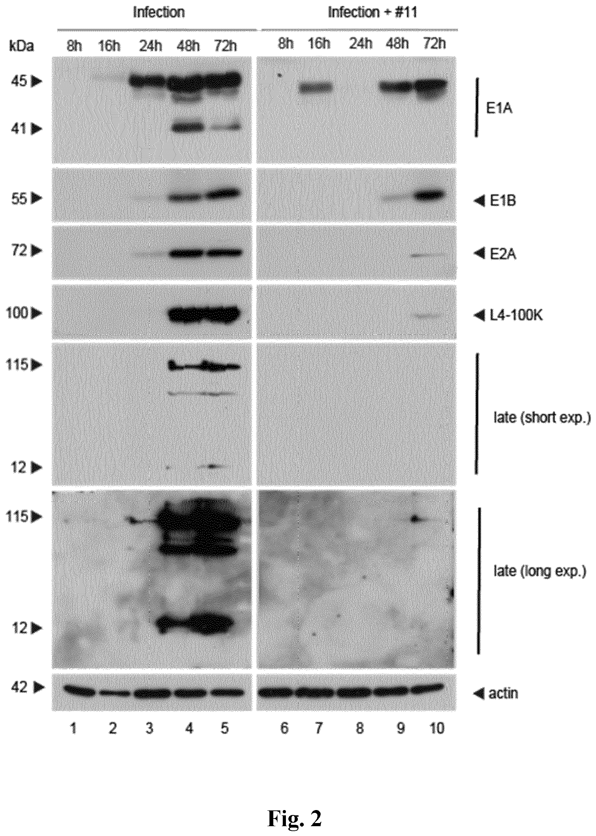

FIG. 2 Western blot of relative expression of adenoviral proteins in A549 cells, infected with HAdV-C5. Left blot: untreated infected cells ("infection"); Right blot: infected cells treated with compound #11 (compound of formula 1a1; "infection + #11"). Right-hand side of the blots: targeted proteins; left-hand side of the blots: molecular weights of the corresponding marker proteins (in kDa).

### EXAMPLES

Out of a total of 30,000 substances that were tested for effectiveness against adenovirus replication in vitro using cell-based high-throughput screening, few candidates with better antiviral activity against HAdVs than the only off-label drug cidofovir used to date were identified. These candidates have high potential to be further developed as clinically used drugs.

### Materials and methods (Ad-inhibitors)

### Cells

A549 cells (ACC 107; DSMZ-German Collection of Microorganisms and Cell Cultures, Braunschweig, Germany) were maintained in DMEM supplemented with 10% foetal bovine serum and 100 U/mL penicillin and 100 µg/mL streptomycin (PAN Biotech). Cells were kept in incubators at 37°C and 5% CO₂. Virus stocks were propagated in A549 cells. All assays were performed with A549 cells.

### Viruses

The HAdV-C5 strain H5pg4100 with deletions in the E3-coding region served as the wt backbone (Groitl, P., and T. Dobner. 2007. 'Construction of adenovirus type 5 early region 1 and 4 virus mutants', Methods Mol Med, 130: 29-39). For the high-throughput screening and follow-up experiments, fluorescent reporter viruses were used. The previously published H5hh4300 was used, which harbours an N-terminally tagged adenovirus DNA-binding protein DBP (mCherry-DBP) (Hidalgo, P., A. Pimentel, D. Mojica-Santamaria, K. von Stromberg, H. Hofmann-Sieber, C. Lona-Arrona, T. Dobner, and R. A. Gonzalez. 2021. 'Evidence That the Adenovirus Single-Stranded DNA Binding Protein Mediates the Assembly of Biomolecular Condensates to Form Viral Replication Compartments', Viruses, 13). Moreover, a virus with a gene encoding the fluorescent protein mNeonGreen was used (Shaner, N. C., G. G. Lambert, A. Chammas, Y. Ni, P. J. Cranfill, M. A. Baird, B. R. Sell, J. R. Allen, R. N. Day, M. Israelsson, M. W. Davidson, and J. Wang. 2013. 'A bright monomeric green fluorescent protein derived from Branchiostoma lanceolatum', Nat Methods, 10: 407-9) fused with DBP (H5hh4313).

Prior to the inhibitor analyses, the virus (designated H5hh4313) was characterized in vitro by growth kinetics, DNA replication, and progeny production as previously described (Boddin J, Ip WH, Wilkens B, von Stromberg K, Ching W, Koyuncu E, Bertzbach LD, Dobner T. 2022. A Single Amino Acid Switch in the Adenoviral DNA Binding Protein Abrogates Replication Center Formation and Productive Viral Infection. mBio 13:e0014422, doi: 10.1128/mbio.00144-22).

To confirm the correct insertion of the transgene, viral DNA was isolated and the target region sequenced by Sanger sequencing.

In all experiments, cells were infected at an MOI (multiplicity of infection) of 1.

### Experimental antiviral compounds and high-throughput screening

In a high-throughput screen, a chemical library containing 30,000 substances was screened for antiadenoviral activity. Two of these preselected substances, namely compounds #11 (compound of formula Ia1; MolPort, Latvia, Riga, www.molport.com) and #15 (compound of formula Ib1; MolPort), and one derivative, #55 (compound of formula Ic1; MolPort), were prioritized for validation. These candidates were initially prepared in 10 mM aliquots in 100% dimethyl sulfoxide (DMSO) and were diluted with DMEM as required.

Positive control: cidofovir (100 µM; hydrate ≥98% (HPLC); MW 279.19 g/mol; Sigma-Aldrich).

### Dose-response curves

Infected and non-infected cells were exposed to varying concentrations of inhibitors in 96-well microtiter plates. Briefly, each well was seeded with 10⁴ cells and incubated overnight. Subsequently, the settled cells were infected, and the target compounds were added at their respective concentrations in each well. Each condition was prepared in quadruplicate with a reaction volume of 100 µL per well, maintaining 1% (v/v) dimethyl sulfoxide (DMSO) in each well. Non-infected cells, treated with equivalent concentrations of DMSO, served as the control. The plates were then incubated for 48 hours.

Following the incubation period, the relative infection and viability of the cells were determined photometrically. The growth medium was removed, and 75 µL of phosphate-buffered saline (PBS) was added. Next, the relative amount of infected cells was estimated through fluorescence measurements using a FLUOstar Omega plate reader (BMG LABTECH) equipped with MARS Data Analysis Software (Version 2.10 R3).

Cell viability was quantified by adding 25 µL of CellTiter-Glo 2.0 reagent (Promega) and measuring luminescence. Following the addition of the CellTiter-Glo 2.0 reagent, the cells were incubated for 10 minutes at room temperature. Then, the supernatants were transferred onto white 96-well microtiter plates, and cell viability was quantified with the FLUOstar Omega plate reader.

### Virus yield assays

The supernatant collected from the infection experiments (8 × 10⁵ infected cells) was introduced to a freshly prepared 96-well plate seeded with A549 cells (reinfection involved diluting the viral titers at a ratio of 1:10). Following incubation periods of 24, 48 and 72 hours, the fluorescence of the plate was assessed using the abovementioned procedure.

### Time-of-addition assays

1 × 10⁴ A549 cells per well were seeded in 96-well microtiter plates in 100 µL of DMEM and incubated overnight at 37°C and 5% CO₂ to allow for cell attachment. Subsequently, the cells were infected and the respective inhibitors administered at defined time intervals post-infection, including a time point two hours before infection to assess any potential inhibition of attachment and entry events.

Following infection, the cells were incubated for approximately 48 hours. Inhibition efficacy and cell viability were quantified through fluorescence and luminescence measurements using the FLUOstar Omega plate reader.

### RT-qPCR

For RT-qPCR analysis, viral DNA was extracted from infected A549 cells harvested at 8, 16, 24, and 48 hours post infection, with untreated infected cells serving as controls.

Viral DNA (vDNA) was isolated from the harvested cells using the QIAamp DNA mini kit (250) (QIAGEN) following the manufacturer's instructions. DNA concentrations were quantified using a NanoDrop1000 (Peqlab).

Subsequently, RT-qPCR analyses were performed using a Corbett Research RG-6000 thermocycler (Qiagen) to determine the relative amounts of viral DNA.

### Immunofluorescence assays

Cells were cultured on glass coverslips and infected 1 day post-seeding. At 48 hpi, the cells were fixed with 4% paraformaldehyde (PFA) for 20 minutes at 4°C, permeabilized in PBS with 0.5% Triton X-100 for 10 minutes at room temperature (RT), and then blocked in Tris-buffered saline with bovine serum albumin and glycine (TBS-BG; 5% wt/vol BSA and 5% wt/vol glycine) for 30 minutes at RT.

Subsequently, coverslips were incubated with the primary antibody diluted in PBS for 1 hour at RT, followed by three washes with TBS-BG. Secondary antibodies, diluted in PBS, were applied to coverslips and incubated for 30 minutes at RT (refer to the antibody table for details). Coverslips were then washed with TBS-BG and PBS and the DNA was stained non-specifically by the addition of 4',6-diamidino-2-phenylindole (DAPI) at a dilution of 1: 1000 in PBS, before mounting using mounting medium (Dako).

Images were acquired using a confocal spinning disk microscope (Nikon Eclipse Ti-E stand; Yokagawa CSU-W1 spinning disk; Andor888 EM-CCD camera; Nikon 100x NA 1.49 objective). Image analysis was performed using Fiji/ImageJ (NIH).

### Western blotting

For protein analysis, cells were suspended in NP-40 lysis buffer (50 mM Tris-HCl [pH 8.0], 150 mM NaCl, 5 mM EDTA, 0.15% Nonidet P-40) supplemented with a protease inhibitor cocktail consisting of 1% (vol/vol) phenylmethylsulfonyl fluoride (PMSF), 0.1% (vol/vol) aprotinin, 1 µg/ml leupeptin, and 1 µg/ml pepstatin. Proteins were boiled 5 min at 95°C in 5 × Laemmli.

For immunoblotting, equal amounts of total protein were separated by SDS-polyacrylamide gel electrophoresis and transferred to nitrocellulose membranes. Membranes were incubated following previously described methods (Kindsmüller, K., S. Schreiner, F. Leinenkugel, P. Groitl, E. Kremmer, and T. Dobner. 2009. 'A 49-kilodalton isoform of the adenovirus type 5 early region 1B 55-kilodalton protein is sufficient to support virus replication', J Virol, 83: 9045-56). The bands were visualized using enhanced chemiluminescence according to the manufacturer's instructions (Pierce) on X-ray films (Super RX-N Fuji Medical X-ray Films; FUJIFILM). Autoradiograms were scanned and edited using Adobe Photoshop.

**Table 1: Primary antibodies**

| | |
|---|---|
| α-actin (45 kDa) | Monoclonal mouse antibody against actin (AC-15), A5441 (Sigma-Aldrich); |
| α-E1A (41-45 kDa) | Monoclonal mouse antibody against HAdV-C5 E1A (M73) (hybridoma antibody) |
| α-E1B-55K (55 kDa) | Monoclonal mouse antibody against the N-terminus of HAdV-C5 E1B-55K (2A6) (Sarnow, Peter, Carole A. Sullivan, and Arnold J. Levine. 1982. 'A monoclonal antibody detecting the adenovirus type 5 E 1 b-58Kd tumor antigen: Characterization of the E 1 b-58Kd tumor antigen in adenovirus-infected and - transformed cells', Virology, 120: 510-17) |
| α-E2A (72 kDa) | Monoclonal mouse antibody against HAdV-C5 E2A (B6-8) (Reich, Nancy C., Peter Sarnow, Elizabeth Duprey, and Arnold J. Levine. 1983. 'Monoclonal antibodies which recognize native and denatured forms of the adenovirus DNA-binding protein', Virology, 128: 480-84) |
| α-capsid (#133) | HAdV-C5 rabbit polyclonal serum (Kzhyshkowska, J., E. Kremmer, M. Hofmann, H. Wolf, and T. Dobner. 2004. 'Protein arginine methylation during lytic adenovirus infection', Biochem J, 383: 259-65) |
| α-L4-100K | Monoclonal rat antibody against HAdV-C5 L4-100K protein (6B10) (Kindsmüller, K., S. Schreiner, F. Leinenkugel, P. Groitl, E. Kremmer, and T. Dobner. 2009. 'A 49-kilodalton isoform of the adenovirus type 5 early region 1B 55-kilodalton protein is sufficient to support virus replication', J Virol, 83: 9045-56) |

**Table 2: Secondary antibodies**

| |
|---|
| Peroxidase-conjugated goat anti-mouse IgG (H+L) (Jackson ImmunoResearch) |
| Peroxidase-conjugated goat anti-rabbit IgG (H+L) (Jackson ImmunoResearch) |
| Peroxidase-conjugated goat anti-rat IgG (H+L) (Jackson ImmunoResearch) |
| Goat anti-rat IgG (H+L), Alexa Fluor 555 (for IF analyses; Invitrogen) |

### Statistical analyses

The dose response curves were prepared in GraphPad PRISM. Data were considered significantly different if p ≤ 0.05.

### Results

Figure 1 shows results of dose-response experiments for compound #11 (compound of formula Ia1). A549 cells were infected with HAdV-C5 at a multiplicity of infection (MOI) of 1 and treated with varying concentrations of compound #11, as indicated. Relative infection levels were compared to two controls: untreated infected cells (positive control) and untreated non-infected cells (negative control). The results indicate inhibition of viral replication and cell viability (shown as an S-curve). The dashed line represents the half-maximal response (50%).

The relative expression of adenoviral proteins was visualized by western blotting (Fig. 2). A549 cells, infected with HAdV-C5 at an MOI of 1, were treated with compound #11 (compound of formula Ia1) and harvested at 8, 16, 24, 48, and 72 hpi (lanes from left to right correspond to different time points for each condition). Untreated infected cells containing 1% (v/v) DMSO were used as the control ('Infection'). The targeted proteins are labeled on the right-hand side of the blots, with the molecular weights of the corresponding marker proteins (in kDa) indicated on the left-hand side. Actin was used as a reference protein. Late HAdV-C5 proteins primarily include capsid proteins such as pVI, pVII, hexon, penton base, and fiber.

Tables 3 and 4 show results of virus titer reduction tests with the above-mentioned anti-adenoviral compounds #11 (compound of formula Ia1), #15 (compound of formula Ib1) and #55 (compound of formula Ic1) in relation to different adenovirus strains.

**Table 3: Titers of adenovirus strains C5, A12, B3 and D37 in the presence of compounds of the invention**

| Inhibitor | C5 | | A12 | | B3 | | D37 | |
|---|---|---|---|---|---|---|---|---|
| 10µM | Mock | Inhibitor | Mock | Inhibitor | Mock | Inhibitor | Mock | Inhibitor |
| #11 | 4,33E+12 | 8,33E+05 | 1,03E+05 | 1,35E+05 | 2,92E+08 | 5,03E+08 | 2,81E+09 | 3,20E+06 |
| #15 | 1, 80E+11 | 5,60E+05 | 3,20E+04 | 5,60E+04 | 3,20E+13 | 3,20E+12 | 5,60E+11 | 5,60E+05 |
| #55 | 9,00E+12 | 1,06E+06 | 2,80E+05 | 8,90E+04 | 3,20E+11 | 3,20E+07 | 1,00E+07 | 5,60E+05 |

**Table 4: Titers of adenovirus strains E4, B14, B14p1 and A31 in the presence of compounds of the invention.**

| Inhibitor | E4 | | B14 | | B14p1 | | A31 | |
|---|---|---|---|---|---|---|---|---|
| 10µM | Mock | Inhibitor | Mock | Inhibitor | Mock | Inhibitor | Mock | Inhibitor |
| #11 | 3,20E+09 | 7,50E+07 | 1,30E+07 | 3,20E+04 | 3,20E+13 | 5,60E+06 | 1,30E+05 | 3,20E+04 |
| #15 | 3,20E+13 | 1,70E+11 | 3,20E+07 | 3,20E+04 | n.d. | n.d. | n.d. | n.d. |
| #55 | 1,00E+13 | 3,20E+09 | n.d. | n.d. | n.d. | n.d. | n.d. | n.d. |

All compounds of the invention show a significant reduction of virus titers in relation to the adenovirus strains tested.

## Claims

1. A chemical compound having the following general formula I 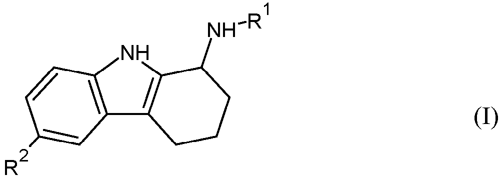 or a salt thereof, wherein
R¹ is selected from the group consisting of the following chemical groups 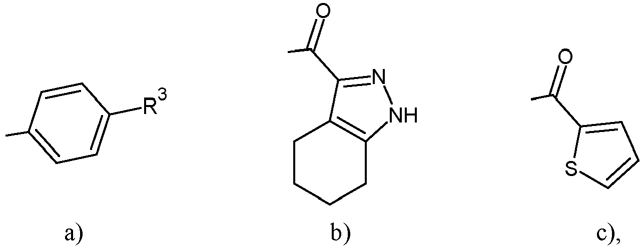
R² is, independently, selected from H, halogen or C₁₋₁₀ alkyl, and
R³ is halogen,
for use as a medicament for treating an adenovirus infection.

2. The chemical compound for use as a medicament for treating an adenovirus infection according to claim 1, wherein
R² is H, halogen, or C₁₋₈ alkyl, preferably C₁₋₆, more preferably C₁₋₄ alkyl, C₁₋₃ alkyl or C₁₋₂ alkyl, most preferably methyl.

3. The chemical compound for use as a medicament for treating an adenovirus infection according to claim 1 or 2, wherein R³ is Br.

4. The chemical compound for use as a medicament for treating an adenovirus infection according to one of the preceding claims, wherein the chemical compound has one of the following general formulas Ia1-Ic1:

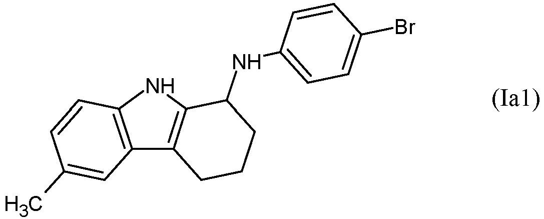

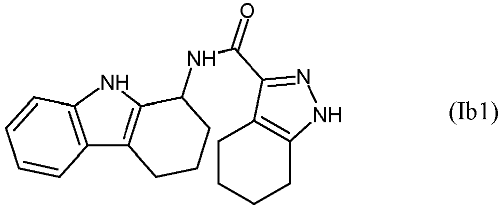

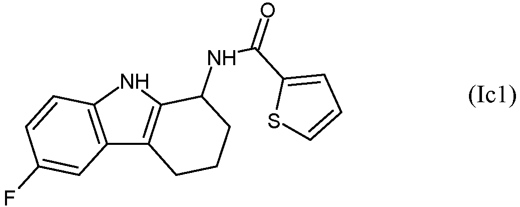

5. A prodrug comprising the compound according to one of the preceding claims for use as a medicament for treating an adenovirus infection.

6. A prodrug according to claim 5, wherein the compound of formula I is N-acylated, N-acyloxyalkylated or N-(phosphoryloxy)alkylated at the N atom of the 1,2,3,4-tetrahydrocarbazole structure of the compound according to formula I.

7. Pharmaceutical composition or dosage form for use as a medicament for treating an adenovirus infection comprising a compound according to one of claims 1 to 4 or a prodrug according to one of claims 5 or 6, and a pharmaceutically acceptable carrier.
